# EUROPEAN PATENT SPECIFICATION

(11) **EP 1 814 886 B1**
(45) Date of publication and mention of the grant of the patent: **22.10.2008**
(21) Application number: 05810971.1
(22) Date of filing: 15.11.2005
(51) Int. Cl.: C07D 495/04

(54) **A PROCESS FOR THE PREPARATION OF N-DEMETHYLOLANZAPINE**
VERFAHREN ZUR HERSTELLUNG VON N-DEMETHYLOLANZAPIN
PROCEDE DE PREPARATION DE N-DEMETHYLOLANZAPINE

(30) Priority: 22.11.2004 PL 37130704
(43) Date of publication of application: 08.08.2007
(60) Divisional application: 08161324.2
(73) Proprietor: Adamed SP. Z O.O., 05-152 Czosnów k/Warszawy (PL)
(72) Inventor: STAWINSKI, Tomasz, PL-05-462 Wiazowna (PL); RECHNIO, Justyna, PL-05-530 Góra Kalwaria (PL); MAJKA, Zbigniew, PL-39-102 Lubzina (PL)
(74) Representative: Sitkowska, Jadwiga
(86) International application number: PCT/EP2005/055981
(87) International publication number: WO 2006/053870

(56) References cited:
- WO-A-20/04000847
- WO-A-20/04094390
- CALLIGARO D O ET AL: "The synthesis and biological activity of some known and putative metabolites of the atypical antipsychotic agent olanzapine (LY170053)" BIOORGANIC & MEDICINAL CHEMISTRY LETTERS, OXFORD, GB, vol. 7, no. 1, 7 January 1997 (1997-01-07), pages 25-30, XP004135960 ISSN: 0960-894X

## Description

The present invention relates to a new process for the preparation of 2-methyl-4-piperazin-1-yl-10H-thieno[2,3-b][1,5]benzodiazepine (hereinafter named N-demethylolanzapine).

N-Demethylolanzapine is useful for the preparation of antipsychotic medicament olanzapine by the process described in WO 2004/000847. Furthermore, N-demethylolanzapine can be useful in therapy, for example of psychoses, anxiety, schizophrenia, and bipolar disease, as described in WO 00/30650.

Known process for the preparation of N-demethylolanzapine as described by Calligaro et al., Bioorg. & Chem. Letters, 1, 25-39 (1997*)*, comprises condensation reaction of 4-amino-2-methyl-10H-thieno[2,3-*b*][1,5]benzodiazepine hydrochloride with anhydrous piperazine. The reaction is carried out in the mixture of high boiling solvents: toluene and DMSO 4:1 (v/v) for several hours, under nitrogen atmosphere.

Organic solvents used in the known process bring serious risks for the environment, worsen the process economics, and the process as a whole is long-lasting, which favors the formation of a product contaminated with by-products.

Furthermore, the solvents used for the preparation of N-demethylolanzapine are difficult to remove and therefore are present in some amounts even in the final product - olanzapine. The removal of solvents is therefore desirable also from the point of view of a patient interest.

It has been unexpectedly found that the condensation reaction of 4-amino-2-methyl-10H-thieno[2,3-*b*][1,5]benzodiazepine with anhydrous piperazine can be carried out in melt in the absence of organic solvents, and that the yield of such process is not lower and may be even improved and the purity of the product is good in comparison with the prior art process.

This is even more surprising in view of that analogous attempts of eliminating solvents (toluene and DMSO mixture) in the condensation reaction of 4-amino-2-methyl-10H-thieno[2,3-b][1,5]benzodiazepine hydrochloride with N-methylpiperazine described in EP 0454436 (the product of which is olanzapine) undertaken by the present inventors in order to diminish environment risk and improve the economics were unsuccessful.

Accordingly, the present invention provides a process for the preparation of N-demethylolanzapine having formula (I): 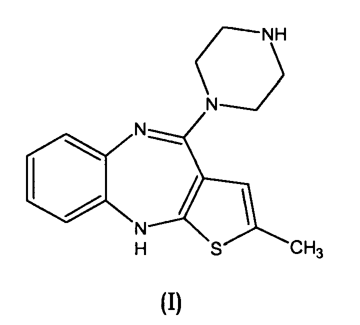 by condensation reaction of piperazine with 4-amino-2-methyl-10H-thieno[2,3-*b*][1,5]benzodiazepine of formula (II) 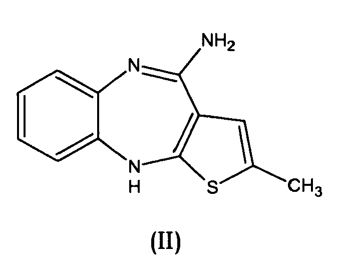 wherein the reaction of anhydrous piperazine with 4-amino-2-methyl-10H-thieno[2,3-*b*][1,5]benzodiazepine or its inorganic acid addition salt is carried out in molten piperazine, in the absence of a solvent.

The reaction is carried out at the temperature above piperazine melting point (m.p.=110-113°C), advantageously at about 130 to 150°C, especially at 150°C.

The reaction may be carried out using both free base form of 4-amino-2-methyl-10H-thieno[2,3-*b*][1,5]benzodiazepine and its addition salt with inorganic acid.

Examples of said salts with inorganic acids are hydrochloride, hydrobromide, sulfate, phosphate, and similar.

Advantageously, for availability reasons, 4-amino-2-methyl-10H-thieno[2,3-*b*][1,5]benzodiazepine may be used in its easy available hydrochloride form.

The reaction can be carried out at piperazine to 4-amino-2-methyl-10H-thieno[2,3-*b*][1,5]benzodiazepine molar ratio in the range from 5:1 to 15:1, preferably from 10:1 do 15:1, especially about 10:1.

Generally, to carry out the process of the invention solid reactants can be combined in a reaction vessel, without using a stirrer, the reaction vessel can be optionally closed, and then the reactants are heated, for example in an oven, up to the piperazine melting point or higher, for sufficient time.

It has been found that 4-amino-2-methyl-10H-thieno[2,3-*b*][1,5]benzodiazepine, especially when in the form of its hydrochloride salt, dissolves very well in molten piperazine to form homogenous mixture. Optimum reaction temperature is about 150°C, at which temperature the reaction takes about 0,5 h. Advantageously, the reaction is carried out in a tightly closed vessel, to prevent piperazine sublimation.

The advantage of the new process both in terms of environment risk and the process economics is elimination of highly boiling solvents and in consequence substantial reduction of wastes and savings in costs of materials.

The process does not require stirring and cooling media both during the reaction and after the reaction to perform crystallization, due to which the process equipment may be simple and the costs substantially lower.

Furthermore, the time of heating is substantially shorter in comparison with the known process. The process of the invention performed at 150°C takes only up to 30 to 45 minutes. In the known process the reaction is carried out for at least 3 hours.

The yield of the process of the invention is very high (up to about 96%).

The operation of recovering of the product from the reaction mixture is very simple and consists in mixing the melt with water at ambient temperature, without cooling, and then separating thus precipitated very fine, easy filterable solid N-demethylolanzapine, for example by filtration. Crude product obtained is of high purity (about 90% HPLC).

N-Demethylolanzapine prepared by the process of the invention may be used for the preparation of olanzapine.

The preparation of olanzapine comprises preparation of N-demethylolanzapine and then N-methylation of N-demethylolanzapine, wherein N-demethylolanzapine is prepared by the process as described above.

N-methylation of N-demethylolanzapine may be performed using known process, for example as described in WO 2004/000847.

The following examples illustrate the invention further without however limiting the scope thereof as presented in the annexed claims.

### EXAMPLES

### Example 1 (comparative)

### Preparation of N-demethylolanzapine by known process (according to Calligaro et al., Bioorg. & Chem. Letters, 1, 25-39 (1997))

A mixture of 4-amino-2-methyl-10H-thieno[2,3-*b*][1,5]benzodiazepine hydrochloride (3 g, 11.3 mmol), piperazine (7 g, 81.4 mmol) in 20 ml of toluene and 5 ml of DMSO was heated to reflux under inert gas atmosphere. The mixture was kept in reflux for 3h. Then the mixture was cooled in an ice/water bath and 30 ml of distilled water was added. The mixture was stirred for 1h at 5°C until the solid formed precipitated completely. Slightly yellow solid precipitate was filtered, washed with water and dried in vacuum desiccator over silica gel. 2.8 g of N-demethylolanzapine with HPLC purity of 91% was obtained (83% yield).

### Example 2

### Preparation of N-demethylolanzapine by the process of the invention

4-Amino-2-methyl-10H-thieno[2,3-*b*][1,5]benzodiazepine hydrochloride (1 g, 3.76 mmol) and anhydrous piperazine (3.24 g, 37.6 mmol) were placed in a tightly closed vessel. The vessel was heated in a laboratory oven at 150°C for 45 minutes. Then the vessel was carefully opened and 20 ml water was poured into the molten mixture. After triturating with water for 2 h, the mixture was filtrated to give a solid. 1.1 g of N-demethylolanzapine of HPLC purity 90% was obtained (96% yield).

### Example 3

### Preparation of N-demethylolanzapine by the process of the invention

In accordance with the procedure of Example 2, the reaction was carried out in the oven at 120°C for 60 minutes. N-demethylolanzapine was obtained with the yield of 80.3% (HPLC purity 88.3%).

### Example 4

### Preparation of N-demethylolanzapine by the process of the invention

In accordance with the procedure of Example 2, the reaction was carried out in the oven at 130°C for 60 minutes. N-demethylolanzapine was obtained with the yield of 80.7% (HPLC purity 87.7%).

### Example 5 (comparative)

### Condensation of 4-amino-2-methyl-10H-thieno[2,3-b][1,5]-benzodiazepine and N-methylpiperazine in melt, without solvent

The reaction of condensation of 4-amino-2-methyl-10H-thieno[2,3-b][1,5]benzodiazepine hydrochloride and N-methylpiperazine at the molar ratio of 1:10 was carried out by heating the reactants in melt at 150°C for 45 minutes. It has been observed that 4-amino-2-methyl-10H-thieno[2,3-b][1,5]benzodiazepine hydrochloride dissolves poorly in N-methylpiperazine, even with heating. The conversion was low. Besides the condensation product (olanzapine), starting material (4-amino-2-methyl-10H-thieno[2,3-b][1,5]benzodiazepine) and substantial amounts of L impurity of the formula: 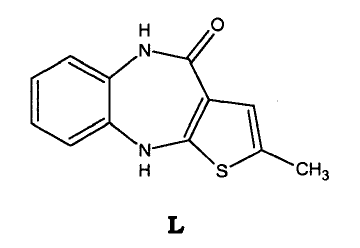 were present in the mixture after the reaction.

The problems were encountered also with the recovery of the product. After addition of water, nonhomogenous gummy precipitate was formed. Because of problems with filtration of the precipitate, extraction of the mixture was necessary. Therefore to ensure homogeneity of the system and simplification of the product recovery it is necessary to carry out the condensation reaction of 4-amino-2-methyl-10H-thieno[2,3-*b*][1,5]benzodiazepine hydrochloride with N-methylpiperazine in the mixture of inert solvents.

## Claims

1. A process for the preparation of N-demethylolanzapine of formula (I) 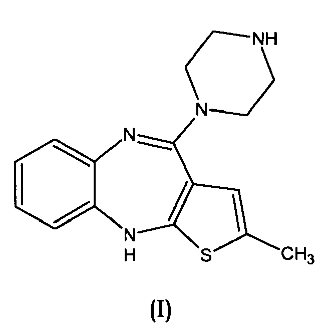 comprising the condensation reaction of piperazine with 4-amino-2-methyl-10H-thieno[2,3-*b*][1,5]benzodiazepine of formula (II) 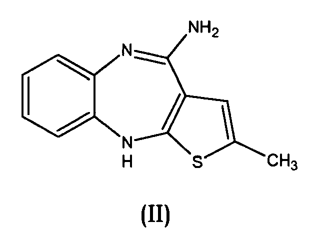 wherein the reaction of anhydrous piperazine with 4-amino-2-methyl-10H-thieno[2,3-*b*][1,5]benzodiazepine or its inorganic acid addition salt is carried out in molten piperazine, in the absence of a solvent.

2. The process of claim 1, wherein the 4-amino-2-methyl-10H-thieno[2,3-*b*][1,5]benzodiazepine salt is hydrochloride.

3. The process of claim 1 or 2, wherein the reaction is carried out at above 110°C.

4. The process of claim 3, wherein the reaction is carried out at about 130 to 150°C, especially about 150°C.

5. The process of claims 1 to 4, wherein the molar ratio of piperazine to 4-amino-2-methyl-10H-thieno[2,3-*b*][1,5]-benzodiazepine is in the range from 5:1 1 to 15:1.

6. The process of claim 5, wherein the molar ratio of piperazine to 4-amino-2-methyl-10H-thieno[2,3-*b*][1,5]-benzodiazepine is in the range from 10:1 to 15:1.

7. The process of claim 5, wherein the molar ratio of piperazine to 4-amino-2-methyl-10H-thieno[2,3-*b*][1,5]-benzodiazepine is about 10:1.

8. The process of claim 1, wherein the reaction of anhydrous piperazine with 4-amino-2-methyl-10H-thieno[2,3-*b*][1,5]benzodiazepine or its hydrochloride is carried out in molten piperazine, in the absence of a solvent, at the molar ratio of piperazine to 4-amino-2-methyl-10H-thieno[2,3-*b*][1,5]benzodiazepine in the range from 5:1 to 15:1, at above 110°C.

9. The process of claim 8, wherein the reaction is carried out at about 130 to 150°C, at the molar ratio of piperazine to 4-amino-2-methyl-10H-thieno[2,3-*b*][1,5]-benzodiazepine in the range from 5:1 to 15:1, especially about 10:1.

10. The process of claim 9, wherein the reaction is carried out at about 150°C.

## Patentansprüche

1. Verfahren zur Herstellung von N-Demethylolanzapin der Formel (I) 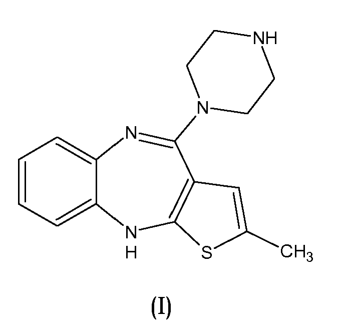 umfassend die Kondensationsreaktion von Piperazin mit 4-Amino-2-methyl-10H-thieno[2,3-b][1,5]benzodiazepin der Formel (II) 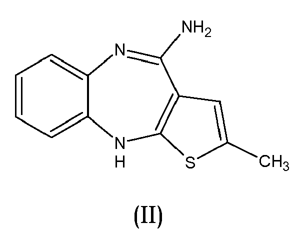 worin die Reaktion von wasserfreiem Piperazin mit 4-Amino-2-methyl-10H-thieno[2,3-*b*][1,5]benzodiazepin oder seinem Additionssalz mit einer anorganischen Säure in geschmolzenem Piperazin in Abwesenheit eines Lösungsmittels durchgeführt wird.

2. Verfahren nach Anspruch 1, worin das Additionssalz von 4-Amino-2-methyl-10H-thieno[2,3-*b*][1,5]benzodiazepin das Hydrochlorid ist.

3. Verfahren nach Anspruch 1 oder 2, worin die Reaktion bei einer Temperatur über 110°C durchgeführt wird.

4. Verfahren nach Anspruch 3, worin die Reaktion bei einer Temperatur von etwa 130 bis 150°C, insbesondere etwa 150°C, durchgeführt wird.

5. Verfahren nach Ansprüchen 1 bis 4, worin das Molverhältnis von Piperazin zu 4-Amino-2-methyl-10H-thieno[2,3-*b*]-[1,5]benzodiazepin im Bereich von 5:1 bis 15:1 liegt.

6. Verfahren nach Anspruch 5, worin das Molverhältnis von Piperazin zu 4-Amino-2-methyl-10H-thieno[2,3-*b*][1,5]benzodiazepine im Bereich von 10:1 bis 15:1 liegt.

7. Verfahren nach Anspruch 5, worin das Molverhältnis von Piperazin zu 4-Amino-2-methyl-10H-thieno[2,3-*b*][1,5]benzodiazepine etwa 10:1 ist.

8. Verfahren nach Anspruch 1, worin die Reaktion von Piperazin mit 4-Amino-2-methyl-10H-thieno[2,3-*b*][1,5]benzodiazepin oder seinem Hydrochlorid in geschmolzenem Piperazin, in Abwesenheit eines Lösungsmittels, bei einem Molverhältnis von Piperazin zu 4-Amino-2-methyl-10H-thieno[2,3-*b*][1,5]benzodiazepin im Bereich von 10:1 bis 15:1, bei einer Temperatur über 110°C durchgeführt wird.

9. Verfahren nach Anspruch 8, worin die Reaktion bei einer Temperatur von etwa 130 bis 150°C, bei einem Molverhältnis von Piperazin zu 4-Amino-2-methyl-10H-thieno[2,3-*b*][1,5]-benzodiazepin im Bereich von 5:1 bis 15:1, insbesondere etwa 10:1, durchgeführt wird.

10. Verfahren nach Anspruch 9, worin die Reaktion bei einer Temperatur etwa 150°C durchgeführt wird.

## Revendications

1. Procédé pour préparation de la N-deméthylolanzapine de formule (I) 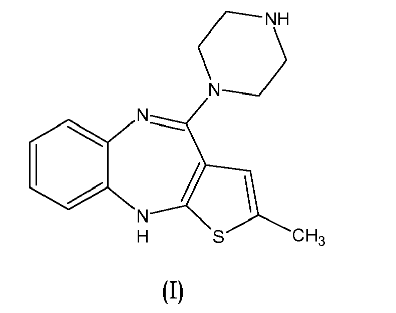 comprenant la réaction de condensation de la pipérazine avec de la 4-amino-2-méthyl-10H-thiéno[2,3-b][1,5]benzodiazépine de formule (II) 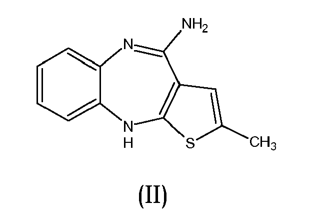 dans lequel la réaction de la pipérazine anhydreé avec de la 4-amino-2-méthyl-10H-thiéno[2,3-b][1,5]benzodiazépine ou d'un sel d'addition d'un acide inorganique de celle-ci est effectuée dans la pipérazine fondue, en absence d'un solvant.

2. Procédé selon la revendication 1, dans lequel la sel de la 4-amino-2-méthyl-10H-thiéno[2,3-b][1,5]benzodiazépine est chlorhydrate.

3. Procédé selon la revendication 1 ou 2, dans lequel la réaction est effectuée à une température au-dessus de 110°C.

4. Procédé selon la revendication 3, dans lequel la réaction est effectuée à une température d'environ 130 à 150°C, en particulier d'environ 150°C.

5. Procédé selon des revendications 1 à 4, dans lequel le rapport molaire de la pipérazine et 4-amino-2-méthyl-10H-thiéno[2,3-b][1,5]benzodiazépine est dans la plage de 5:1 à 15:1.

6. Procédé selon la revendication 5, dans lequel le rapport molaire de la pipérazine et 4-amino-2-méthyl-10H-thiéno[2,3-b][1,5]benzodiazépine est dans la plage de 10:1 à 15:1.

7. Procédé selon la revendication 5, dans lequel le rapport molaire de la pipérazine et 4-amino-2-méthyl-10H-thiéno[2,3-*b*][1,5]benzodiazépine est d'environ 10:1.

8. Procédé selon la revendication 1, dans lequel la réaction de la pipérazine anhydreé avec de la 4-amino-2-méthyl-10H-thiéno[2,3-b][1,5]benzodiazépine ou son chlorhydrate est effectuée dans la pipérazine fondue, en absence d'un solvant, à un rapport molaire de la pipérazine et 4-amino-2-méthyl-10H-thiéno[2,3-*b*][1,5]benzodiazépine etant dans la plage de 5:1 à 15:1, et à une température au-dessus de 110°C.

9. Procédé selon la revendication 8, dans lequel la réaction est effectuée à une température d'environ 130 à 150°C, à un rapport molaire de la pipérazine et 4-amino-2-méthyl-10H-thiéno[2,3-*b*][1,5]benzodiazépine etant dans la plage de 5:1 à 15:1, en particulier d'environ 10:1.

10. Procédé selon la revendication 9, dans lequel la réaction est effectuée à une température d'environ 150°C.
